# EUROPÄISCHE PATENTSCHRIFT

(11) **EP 2 931 224 B1**
(45) Veröffentlichungstag und Bekanntmachung des Hinweises auf die Patenterteilung: **28.06.2017**
(21) Anmeldenummer: 13789009.1
(22) Anmeldetag: 11.11.2013
(51) Int. Cl.: A61K 8/31, A61K 8/34, A61K 8/58, A61Q 15/00, A61K 8/26, A61K 8/28, A45D 40/00, A61K 8/89, A61K 8/92, A61K 8/19

(54) **WASSERFREIE ZUSAMMENSETZUNGEN GEGEN KÖRPERGERUCH**
ANHYDROUS COMPOSITIONS ACTIVE AGAINST BODY ODOURS
COMPOSITIONS ANHYDRES CONTRE LES ODEURS CORPORELLES

(30) Priorität: 14.12.2012 DE 102012223197
(43) Veröffentlichungstag der Anmeldung: 21.10.2015
(73) Patentinhaber: Henkel AG & Co. KGaA, 40589 Düsseldorf (DE)
(72) Erfinder: DÖRING, Thomas, 41540 Dormagen (DE)
(86) Internationale Anmeldenummer: PCT/EP2013/073471
(87) Internationale Veröffentlichungsnummer: WO 2014/090491

(56) Entgegenhaltungen:
- EP-A1- 2 189 149
- WO-A2-02/087516
- DE-C1- 4 223 871
- US-A- 4 725 432
- US-A1- 2003 202 949
- US-A1- 2004 081 632

## Beschreibung

Die vorliegende Erfindung betrifft Kosmetika gegen Körpergeruch, insbesondere wasserfreie Zusammensetzungen, die als Nonaerosol appliziert werden.

Zusammensetzungen gegen Körpergeruch sind ein wichtiger Bestandteil der täglichen persönlichen Hygiene. Sie sollen dafür sorgen, dass der im Laufe des Tages durch diverse Aktivitäten (körperliche Bewegung, Arbeit, Sport), aber auch durch psychische Belastung gebildete Schweiß nicht zu unangenehmem Körpergeruch führt. Genau so vielfältig, wie die Bestandteile des Schweißes und die Ursachen für die Körpergeruchsentwicklung sind, sind auch die deodorierenden Wirkstoffe der handelsüblichen Deodorantien. Als kosmetische deodorierende Wirkstoffe einsetzbar sind Geruchsabsorber, Duftstoffe, desodorierend wirkende lonenaustauscher, keimhemmende Mittel, präbiotisch wirksame Komponenten sowie Enzyminhibitoren. Körpergeruch ist, vereinfacht dargestellt, auf die bakterielle Zersetzung der organischen Bestandteile des Schweißes zurückzuführen. Für die bakterielle Zersetzung wiederum sind einige der für die natürliche Mikroflora der menschlichen Haut typischen Bakterien verantwortlich, insbesondere Gram-positive anaereobe Kokken, z. B. Staphylococcen, wie Staphylococcus hominis, und Corynebakterien. Da der Körpergeruch durch bakterielle Tätigkeit entsteht, kann er besonders wirksam durch die Anwendung kosmetischer Mittel (Seifen, Cremes, Puder, Stifte, Roller, Gele oder Sprays) verhindert werden, die antimikrobiell wirksame Stoffe und Parfümölkompositionen enthalten. Als antimikrobiell wirksame Stoffe werden zur Herstellung von Desodorantien unter anderen Triclosan und Chlorhexidine verwendet. Weiter werden als Wirkstoffe für Desodorantien ätherische Öle wie Nelkenöl (Eugenol), Pfefferminzöl (Menthol) und Thymianöl (Thymol) verwendet, wobei der ausgeprägte Eigengeruch dieser Verbindungen jedoch dosislimitierend wirkt. Als chlororganische Verbindung ist Triclosan ökologisch nicht unumstritten. Ätherische Öle haben ein relativ hohes allergenes Potenzial, was, zusätzlich zu ihrem Eigengeruch, ihren Einsatz einschränkt. Aromatische Alkohole, wie beispielsweise 2-Methyl-5-phenyl-pentan-1-ol, 2-Phenylethan-l-ol oder 3-Phenylpropan-1-ol, sind im Stand der Technik bereits als antimikrobielle und deodorierende Wirkstoffe bekannt. Diese Verbindungen sind hoch wirksam, zugleich sehr gut hautverträglich, und weisen nur einen schwachen Eigengeruch auf.

Als Applikationsformen für besagte Zusammensetzungen haben sich das Aerosolspray, der Roll-on sowie Antitranspirans-Sticks am Markt etabliert. Ferner sind auch das Deodorant in Puderform (auch als kompaktiertes Puder) oder das auf einem wegwerfbaren Substrat (wie einem Tuch, Pad oder Bausch) aufgebrachte Deodorant, bekannt. Als besonders angenehme Anwendungsform kennt der Fachmann die sogenannten weichen Feststoffe ("soft solids"). Darunter werden viskose Zusammensetzungen verstanden, die eine cremige Textur besitzen und vor der Anwendung durch ein oder mehrere Öffnungen einer Abgabevorrichtung des Applikators herausgedrückt werden. Bei diesem Vorgang wird ein Druck auf die Zusammensetzung ausgeübt, unter dem die Rezeptur oftmals instabil wird und dabei einen seiner flüssigen Bestandteile abscheidet. Dieses Phänomen wird Synerese genannt und ist meist bei soft solids mit hohem Ölgehalt zu beobachten.

Aufgabe der vorliegenden Anmeldung war es daher, eine deodorierende kosmetische Zusammensetzung in Form einer wasserfreien Zusammensetzung als soft solid bereitzustellen, die keine oder zumindest eine verminderte Synerese aufweisen. Darüber hinaus soll die Zusammensetzung auf der Haut ein leichtes, puderiges und trockenes Gefühl vermitteln. Wenn die Zusammensetzung mit Textilien in Kontakt tritt, soll die Zusammensetzung leicht aus den Textilien auszuwaschen sein.

Es wurde gefunden, dass die gestellte Aufgabe durch folgenden ersten Erfindungsgegenstand gelöst wird: Wasserfreie Zusammensetzungen, die jeweils bezogen auf das Gesamtgewicht der Zusammensetzung enthalten
- mindestens einen Antitranspirant-Wirkstoff,
- mindestens ein Öl in einer Gesamtmenge von 20 bis 80 Gew.-%,
- mindestens einen (insbesondere festen) Fettalkohol mit 12 bis 18 Kohlenstoffatomen,
- mindestens einen (insbesondere festen) Fettalkohol mit mehr als 18 Kohlenstoffatomen,
mit der Maßgabe, dass
- die Gesamtmenge an (insbesondere festen) Fettalkoholen 3 bis 12 Gew.-% beträgt,
- das Gewichtsverhältnis der (insbesondere festen) Fettalkohole mit 12 bis 18 Kohlenstoffatomen zu den übrigen (insbesondere festen) Fettalkoholen in einem Gewichtsverhältnisbereich von 1 zu 1 bis 1 zu 1,3 liegt.

Unter Fettalkoholen werden erfindungsgemäß primäre Alkohole in Form von Monohydroxyverbindungen eines linearen, aliphatischen Kohlenwasserstoffs verstanden, wobei der besagte Kohlenwasserstoff wenigstens 12 Kohlenstoffatome aufweist und außer der Hydroxygruppe keinen weiteren Substituenten trägt.

Alle Angaben über die Aggregatzustände der verwendeten Ausgangsstoffe (fest, flüssig...) in dieser Anmeldung beziehen sich auf Normalbedingungen. "Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Der Begriff "wasserfrei" wird erfindungsgemäß so verstanden, dass die Zusammensetzungen 0 bis maximal 3 Gew.-%, bevorzugt 0 bis maximal 2 Gew.-%, freies Wasser enthalten, bezogen auf die gesamte Zusammensetzung. Der Gehalt an Kristallwasser, Hydratationswasser oder ähnlich molekular gebundenem Wasser, der in den eingesetzten Bestandteilen, insbesondere in gegebenenfalls enthaltenen schweißhemmenden Wirkstoffen, enthalten sein kann, stellt im Sinne der vorliegenden Anmeldung kein freies Wasser dar.

Die erfindungsgemäßen Zusammensetzungen enthalten zwingend - zur Unterstützung der Gesamtdeodorant-Leistung des Produktes - mindestens einen schweißhemmenden Wirkstoff, der auch als Antitranspirant-Wirkstoff bezeichnet wird.

Besonders bevorzugte erfindungsgemäße Zusammensetzungen sind dadurch gekennzeichnet, dass der mindestens eine Antitranspirant-Wirkstoff in einer Menge von 3 bis 35 Gew.-%, bevorzugt 5 bis 30 Gew.-% und besonders bevorzugt 10 bis 23 Gew.-%, enthalten ist, bezogen auf das Gesamtgewicht der kristallwasserfreien Aktivsubstanz (USP) in der Gesamtzusammensetzung.

Bevorzugte Antitranspirant-Wirkstoffe sind ausgewählt aus den wasserlöslichen adstringierenden anorganischen und organischen Salzen des Aluminiums, Zirkoniums und Zinks bzw. beliebigen Mischungen dieser Salze.

Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffs in 95 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl₂ · 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorhydrex-Propylenglykol (PG) oder Aluminiumchlorhydrex-Polyethylenglykol (PEG), Aluminium- oder Aluminiumzirkonium-Glycol-Komplexe, z. B. Aluminium- oder Aluminiumzirkonium-Propylenglycol-Komplexe, Aluminiumsesquichlorhydrex-PG oder Aluminiumsesquichlorhydrex-PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexen von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat, Zirconyloxyhalogeniden, insbesondere Zirconyloxychloriden, Zirconylhydroxyhalogeniden, insbesondere Zirconylhydroxychloriden (Zirkoniumchlorohydrat).

Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminium- und Aluminium-Zirconiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminium- und Aluminium-Zirconiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminium- und Aluminium-Zirconiumsalze müssen nicht notwendigerweise als sprühgetrocknetes Pulver eingesetzt werden. Erfindungsgemäß ebenfalls bevorzugte schweißhemmende Wirkstoffe sind nicht-wässrige Lösungen oder Solubilisate eines aktivierten schweißhemmenden Aluminium- oder Aluminium-Zirconiumsalzes, beispielsweise gemäß US 6010688, die durch den Zusatz einer wirksamen Menge eines mehrwertigen Alkohols, der 3 bis 6 Kohlenstoffatome und 3 bis 6 Hydroxyl-Gruppen, bevorzugt Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18 - 45 Gew.-% eines aktivierten Aluminium- oder Aluminium-Zirconiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt Propylenglycol, Butylenglycol, Diethylenglycol, Dipropylenglycol, Glycerin, Sorbit und Pentaerythrit, besonders bevorzugt Propylenglycol.

Besonders bevorzugt sind auch Komplexe aktivierter schweißhemmender Aluminium- oder Aluminium-Zirconiumsalze mit einem mehrwertigen Alkohol, die 20 - 50 Gew.-%, besonders bevorzugt 20 - 42 Gew.-%, aktiviertes schweißhemmendes Aluminium- oder Aluminium-Zirconiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminium- oder Aluminium-Zirconiumsalzes mit einem mehrwertigen Alkohol sind z. B. offenbart in US 5643558 und US 6245325.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, wie sie beispielsweise in US 4017599 offenbart sind, die mit Salzen von Aminosäuren, insbesondere mit Alkali- und Erdalkaliglycinaten, gepuffert sind.

Weitere bevorzugte schweißhemmende Wirkstoffe sind aktivierte Aluminium- oder Aluminium-Zirconiumsalze, wie sie beispielsweise in US 6245325 oder US 6042816 offenbart sind, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Calciumsalz in einer solchen Menge, um ein Ca:(AI+Zr)-Gewichtsverhältnis von 1:1 - 1:28 und bevorzugt 1:2 - 1:25 bereitzustellen. Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(AI+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:(AI+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Calciumsalz, dass das Ca:(AI+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der l-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminium- oder Aluminium-Zirconiumsalze, wie sie beispielsweise in US 6902723 offenbart sind, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Strontiumsalz in einer solchen Menge, um ein Sr:(AI+Zr)-Gewichtsverhältnis von 1:1 - 1:28 und bevorzugt 1:2 - 1:25 bereitzustellen.

Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(AI+Zr)-Gewichtsverhältnis 1:1 - 1:28, bevorzugt 1:2 - 1:25, beträgt und soviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(AI+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser, weiterhin soviel wasserlösliches Strontiumsalz, dass das Sr:(AI+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Bevorzugte aktivierte Aluminium-Zirconiumsalze sind solche, die Mischungen oder Komplexe der vorstehend beschriebenen Aluminiumsalze mit Zirconiumsalzen der Formel ZrO(OH)_{2-pb}Y_{b} darstellen, worin Y Cl, Br, I, NO₃ oder SO₄ ist, b eine rationale Zahl von 0,8 bis 2 und p die Wertigkeit von Y ist, wie sie beispielsweise in US 6074632 offenbart sind. Die Zirconiumsalze haben in der Regel ebenfalls etwas Hydratationswasser assoziativ gebunden, typischerweise 1 bis 7 Mol Wasser pro Mol Salz. Vorzugsweise ist das Zirconiumsalz Zirconylhydroxychlorid mit der Formel ZrO(OH)_{2-b}Cl_{b}, worin b eine rationale Zahl von 0,8 bis 2, bevorzugt 1,0 bis 1,9 ist. Bevorzugte Aluminium-Zirconiumsalze haben ein AI:Zr-Molverhältnis von 2 bis 10 und ein Metall:(X+Y)-Verhältnis von 0,73 bis 2,1, bevorzugt 0,9 bis 1,5. Ein besonders bevorzugtes Salz ist Aluminium-Zirconiumchlorhydrat (d.h., X und Y sind Cl), das ein AI:Zr-Verhältnis von 2 bis 10 und ein molares Metall:Cl-Verhältnis von 0,9 bis 2,1 hat. Der Begriff Aluminium-Zirconiumchlorhydrat umfasst die Tri-, Tetra-, Penta- und Octachlorhydratformen.

Erfindungsgemäß bevorzugte Zirconiumsalze haben die allgemeine Formel ZrO(OH)₂₋ₐClₐ · x H₂O mit a = 1.5 - 1.87; x = 1 - 7, wobei a und x rationale Zahlen sind. Diese Zirconiumsalze sind beispielsweise in der belgischen Schrift BE 825146 offenbart.

Weitere bevorzugte schweißhemmende Wirkstoffe sind in US 6663854 und US 20040009133 offenbart.

Die schweißhemmenden Wirkstoffe können sowohl in solubilisierter als auch in ungelöster, suspendierter Form vorliegen.

Sofern die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen.

Bevorzugte Aluminiumsalze und Aluminiumzirconiumsalze weisen ein molares Metall-zu-Chlorid-Verhältnis von 0,9 - 1,3, bevorzugt 0,9 - 1,1, besonders bevorzugt 0,9 - 1,0, auf.

Bevorzugte Aluminiumzirconiumchlorohydrate haben im allgemeinen die empirische Formel AlₙZr(OH)_{[3n+4-m(n+1)]}(Cl)_{[m(n+1)]} mit n = 2,0 - 10,0, bevorzugt 3,0 - 8,.0, m = 0,77 - 1,11 (entsprechend einem molaren Metall (AI+Zr)-zu-Chlorid-Verhältnis von 1,3 - 0,9), bevorzugt m = 0,91 - 1,11 (entsprechend M:CI = 1,1 - 0,9), und besonders bevorzugt m = 1,00 - 1,11 (entsprechend M:CI = 1,0 - 0,9), weiterhin sehr bevorzugt m = 1,02 - 1,11 (entsprechend M:CI = 0,98 - 0,9) sowie sehr bevorzugt m = 1,04 - 1,11 (entsprechend M:CI = 0,96 - 0,9).

Bei diesen Salzen ist im Allgemeinen etwas Hydratationswasser assoziativ gebunden, typischerweise 1 - 6 Mol Wasser pro Mol Salz, entsprechend 1 - 16 Gew.-%, bevorzugt 4 - 13 Gew.-% Hydratationswasser.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der I-Form und der dl-Form; Glycin ist besonders bevorzugt. Die Aminosäure ist in einer Menge von 1 - 3 Mol, bevorzugt 1,3 - 1,8 Mol, jeweils pro Mol Zirconium in dem Salz enthalten.

Bevorzugte schweißhemmende Salze sind Aluminium-Zirconiumtetrachlorohydrat (Al:Zr = 2-6; M:CI = 0.9-1.3), insbesondere Salze mit einem molaren Metall-zu-Chlorid-Verhältnis von 0,9 - 1,1, bevorzugt 0,9 - 1,0.

Weiterhin erfindungsgemäß bevorzugt sind Aluminiumzirconiumchlorohydrat-Glycin-Salze, die mit Betain ((CH₃)₃N⁺-CH₂-COO⁻) stabilisiert sind. Besonders bevorzugte entsprechende Verbindungen weisen ein molares Gesamt-(Betain + Glycin)/Zr-Verhältnis von (0,1 - 3,0): 1, bevorzugt (0,7 - 1,5) : 1 und ein molares Verhältnis von Betain zu Glycin von mindestens 0,001 : 1 auf. Entsprechende Verbindungen sind beispielsweise offenbart in US 7105691.

In einer besonders bevorzugten erfindungsgemäßen Ausführungsform ist als besonders wirksames Antitranspirant-Salz ein so genanntes "aktiviertes" Salz enthalten, insbesondere eines mit einem hohen HPLC-Peak 5-Aluminium-Gehalt, insbesondere mit einer Peak 5-Fläche von mindestens 33 %, besonders bevorzugt mindestens 45 %, bezogen auf die gesamte Fläche unter den Peaks 2 - 5, gemessen mit HPLC einer 10 Gew.-%igen wässrigen Lösung des Wirkstoffs unter Bedingungen, bei denen die Aluminiumspecies in mindestens 4 aufeinander folgende Peaks aufgelöst werden (mit Peaks 2 - 5 bezeichnet). Bevorzugte Aluminiumzirconiumsalze mit einem hohen HPLC-Peak 5-Aluminium-Gehalt (auch als "E⁵AZCH" bezeichnet) sind beispielsweise offenbart in US 6436381 und US 6649152.

Weiterhin sind solche aktivierten "E⁵AZCH"-Salze bevorzugt, deren HPLC-Peak 4-zu-Peak 3-Flächenverhältnis von mindestens 0,4, bevorzugt mindestens 0,7, besonders bevorzugt mindestens 0,9, beträgt.

Weitere besonders bevorzugte Antitranspirant-Wirkstoffe sind solche Aluminiumzirconiumsalze mit einem hohen HPLC-Peak 5-Aluminium-Gehalt, die zusätzlich mit einem wasserlöslichen Strontiumsalz und/oder mit einem wasserlöslichen Calciumsalz stabilisiert sind. Entsprechende Salze sind beispielsweise in US 6923952 offenbart.

Weitere bevorzugte Antitranspirant-Wirkstoffe sind ausgewählt aus adstringierenden Titansalzen, wie sie beispielsweise in GB 2299506 A offenbart sind.

Die Antitranspirant-Wirkstoffe können als nicht-wässrige Lösungen oder als glycolische Solubilisate eingesetzt werden.

In einer besonders bevorzugten Ausführungsform enthält die Zusammensetzung ein adstringierendes Aluminiumsalz, insbesondere Aluminiumchlorohydrat, das beispielsweise pulverförmig als Micro Dry^{®} Ultrafine oder Superultrafine von Reheis, Microdry 323 von Summit, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten, das ebenfalls besonders bevorzugt ist. Besonders bevorzugt sind auch aktivierte Aluminiumchlorohydrate, die unter den Bezeichnungen Reach^{®} 101 und Reach^{®} 103 , AACH- 7171 von Reheis oder Summit erhältlich sind. Auch die Verwendung von Aluminium-Zirkonium-Tetrachlorohydrex-Glycin-Komplexen, die beispielsweise von Reheis unter der Bezeichnung Rezal^{®} 36 GP von Reheis oder AZG-364 oder 369 von Summit, in aktivierter Qualität, als Reach^{®} 908, als Pulver im Handel sind, kann erfindungsgemäß besonders bevorzugt sein. Auch Aluminium-Zirkoniumpentachlorohydex- Glycin-Komplexe (AAZG-3108 oder AAZG-3110 von Summit) können zum Einsatz kommen.

Die erfindungsgemäße Zusammensetzung enthält weiterhin zwingend mindestens ein Öl in besagter Gesamtmenge. Unter einem Öl ist erfindungsgemäß ein flüssiger Stoff zu verstehen, der bei Normalbedingungen in bidestilliertem Wasser zu weniger als 1 Gew.-% mischbar ist.

Es ist erfindungsgemäß bevorzugt, wenn die erfindungsgemäße Zusammensetzung bezogen auf das Gewicht der Zusammensetzung Öle in einer Gesamtmenge von 30 bis 80 Gew.-%, besonders bevorzugt von 40 bis 75 Gew.-%, ganz besonders bevorzugt von 45 bis 70 Gew.%, in der erfindungsgemäßen Zusammensetzung enthalten sind.

Besonders bevorzugt enthält die erfindungsgemäße Zusammensetzung als Öl mindestens ein flüchtiges Öl. Dabei ist es wiederum bevorzugt, wenn die flüchtigen Öle bezogen auf das Gewicht der Zusammensetzung in einer Gesamtmenge von 30 bis 80 Gew.-%, besonders bevorzugt von 40 bis 75 Gew.-%, ganz besonders bevorzugt von 45 bis 70 Gew.-%, in der erfindungsgemäßen Zusammensetzung enthalten sind.

Als flüchtiges Öl werden erfindungsgemäß Öle verstanden, die bei 293,15 K einen Dampfdruck von 0,01 kPa oder mehr aufweisen.

Erfindungsgemäß bevorzugte Öle sind ausgewählt aus Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen.

Besonders bevorzugte flüchtige Öle sind flüchtige Silikonöle und flüchtige Nichtsilikonöle. Flüchtige Siliconöle, die cyclisch sein können, wie z. B. Octamethylcyclotetrasiloxan, Decamethylcyclopentasiloxan sowie Mischungen hiervon, wie sie z. B. in den Handelsprodukten DC 244, 245, 344 und 345 von Dow Corning enthalten sind, sind erfindungsgemäß bevorzugt. Ebenfalls besonders bevorzugt sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/oder L₄, bevorzugt solche Mischungen, wie sie z. B. in den Handelsprodukten DC 2-1184, Dow Corning^{®} 200 (0,65 cSt) und Dow Corning^{®} 200 (1,5 cSt) von Dow Corning enthalten sind.

Flüchtige Siliconöle sind erfindungsgemäß hervorragend geeignet, da sie der erfindungsgemäßen Zusammensetzung ein angenehmes Hautgefühl und eine geringe Kleideranschmutzung verleihen. Erfindungsgemäß besonders bevorzugte Zusammensetzungen sind daher durch einen Gehalt an mindestens einem flüchtigen Siliconöl gekennzeichnet. Dabei ist es wiederum bevorzugt, wenn die flüchtigen Silikonöle bezogen auf das Gewicht der Zusammensetzung in einer Gesamtmenge von 20 bis 80 Gew.-%, insbesondere von 30 bis 80 Gew.-%, besonders bevorzugt von 40 bis 75 Gew.-%, ganz besonders bevorzugt von 45 bis 70 Gew.-%, in der erfindungsgemäßen Zusammensetzung enthalten sind.

Neben oder anstelle des mindestens einen flüchtigen Siliconöls kann auch mindestens ein flüchtiges Nichtsiliconöl enthalten sein. Bevorzugte flüchtige Nichtsiliconöle sind ausgewählt aus C₈-C₁₆-Isoparaffinen, insbesondere aus Isodecan, Isododecan, Isotetradecan und Isohexadecan sowie Mischungen hiervon. Insbesondere eignet sich bevorzugt C10-13 Isoparaffin (z.B. Handelsprodukt Pioner 2094 von Hansen & Rosenthal).

Auch dieses mindestens eine flüchtige Nichtsiliconöl ist bevorzugt in einer Gesamtmenge von von 20 bis 80 Gew.-%, besonders bevorzugt von 30 bis 80 Gew.-%, ganz besonders bevorzugt von 40 bis 75 Gew.-%, jeweils bezogen auf das Gesamtgewicht der Zusammensetzung, enthalten.

Aufgrund des Hautgefühls und der Stabilität der resultierenden Zusammensetzungen sind Siliconöle als flüchtiges Öl gegenüber Isoparaffinen besonders bevorzugt.

Neben den vorgenannten, üblicherweise als flüchtigen Siliconölen bezeichneten Substanzen sowie neben den vorgenannten flüchtigen Nichtsiliconölen können die erfindungsgemäßen Zusammensetzungen zusätzlich mindestens ein nichtflüchtiges Öl, ausgewählt aus nichtflüchtigen Siliconölen und nichtflüchtigen Nichtsiliconölen, enthalten.

Bevorzugte nichtflüchtige Siliconöle sind ausgewählt aus höhermolekularen linearen Dimethylpolysiloxanen, im Handel erhältlich z. B. unter der Bezeichnung Dow Corning^{®} 190, Dow Corning^{®} 200 Fluid mit kinematischen Viskositäten (25°C) im Bereich von 5 - 100 cSt, bevorzugt 5 - 50 cSt oder auch 5 - 10 cSt, und Baysilon^{®} 350 M (mit einer kinematischen Viskosität (25°C) von etwa 350 cSt.

Erfindungsgemäß ebenfalls bevorzugte nichtflüchtige Siliconöle sind ausgewählt aus Siliconen der Formel (Sil-1), wobei x ausgewählt ist aus ganzen Zahlen von 1 - 20, bevorzugt 1 - 3.

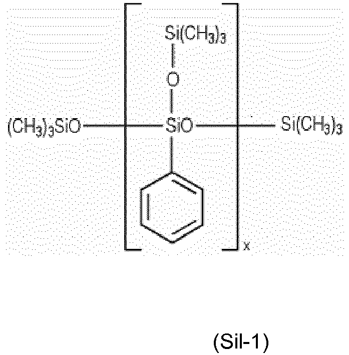

Ein bevorzugtes Siliconöl der Formel (Sil-1) ist erhältlich unter der INCI-Bezeichnung Phenyl Trimethicone.

Natürliche und synthetische Kohlenwasserstoffe, wie beispielsweise Paraffinöle, C₁₈-C₃₀-Isoparaffine, insbesondere Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (erhältlich z. B. unter dem Handelsnamen Cetiol^{®}S von Cognis) gehören ebenfalls zu den erfindungsgemäß bevorzugten nichtflüchtigen Nichtsiliconölen.

Weitere erfindungsgemäß bevorzugte nichtflüchtige Nichtsiliconöle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD.

Weitere erfindungsgemäß bevorzugte nichtflüchtige Nichtsiliconöle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltriisostearin und die Handelsprodukte Estol^{®} GTEH 3609 (Uniqema) oder Myritol^{®} GTEH (Cognis) mit verzweigten Fettsäureresten.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Estern der linearen oder verzweigten gesättigten oder nichtflüchtige Nichtsiliconöle, Ester ungesättigter Alkanole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Hexyldecylstearat (Eutanol^{®} G 16 S), Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).

Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether (Ucon Fluid^{®} AP), PPG-9-Butylether (Breox^{®} B25), PPG-10-Butandiol (Macol^{®} 57), PPG-15-Stearylether (Arlamol^{®} E) und Glycereth-7-diisononanoat.

Weitere erfindungsgemäß besonders bevorzugte nichtflüchtige Nichtsiliconöle sind ausgewählt aus den C₈-C₂₂-Alkanolestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂₋C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI.

Weitere erfindungsgemäß besonders bevorzugte nichtflüchtige Nichtsiliconöle sind ausgewählt aus den symmetrischen, unsymmetrischen oder cyclischen Estern der Kohlensäure mit Alkanolen, z. B. Glycerincarbonat, Dicaprylylcarbonat (Cetiol^{®} CC) oder die Ester gemäß der Lehre der DE 19756454 A1.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Es ist erfindungsgemäß bevorzugt, wenn die erfindungsgemäßen Zusammensetzungen bezogen auf das Gesamtgewicht der Zusammensetzung die nichtflüchtigen Öle in einer Gesamtmenge von 0 bis 20 Gew.-%, insbesondere von 0 bis 10 Gew.-% enthalten.

Die erfindungsgemäße Zusammensetzung enthält zwingend eine definierte Mischung von Fettalkoholen.

Es ist erfindungsgemäß bevorzugt, wenn die zur Herstellung der erfindungsgemäßen Zusammensetzung verwendeten Fettalkohole als Feststoff vorliegen und sich alle auf Fettalkohole beziehenden Definitionen auf feste Fettalkohole im Sinne der Anmeldung beziehen und flüssige Fettalkohole ausschließlich der Ölkomponente der erfindungsgemäßen Zusammensetzung zugerechnet werden.

In einer erfindungsgemäß bevorzugten Zusammensetzung beträgt bezogen auf das Gesamtgewicht der Zusammensetzung die Gesamtmenge an Fettalkoholen 4 bis 9 Gew.-%.

Es ist erfindungsgemäß bevorzugt, wenn die Zusammensetzung bezogen auf ihr Gesamtgewicht die Fettalkohole mit 18 Kohlenstoffatomen in einer Gesamtmenge von 2,0 bis 5,0 Gew.-% enthält.

Es ist erfindungsgemäß bevorzugt, wenn die Zusammensetzung bezogen auf ihr Gesamtgewicht die Fettalkohole mit 20 bis 28 Kohlenstoffatomen in einer Gesamtmenge von 0,8 bis 2,0 Gew.-% enthält.

Es ist erfindungsgemäß bevorzugt, wenn die Zusammensetzung bezogen auf ihr Gesamtgewicht die Fettalkohole mit 30 bis 38 Kohlenstoffatomen in einer Gesamtmenge von 0,6 bis 1,5 Gew.-% enthält.

Es ist erfindungsgemäß bevorzugt, wenn die Zusammensetzung bezogen auf ihr Gesamtgewicht die Fettalkohole mit 40 bis 48 Kohlenstoffatomen in einer Gesamtmenge von 0,2 bis 0,7 Gew.-% enthält.

Davon zunächst unabhängig ist es bevorzugt, wenn die erfindungsgemäße Zusammensetzung bezogen auf ihr Gesamtgewicht weniger als 0,05 Gew.-% Fettalkohole mit mehr als 50 Kohlenstoffatomen enthält.

Davon zunächst unabhängig ist es bevorzugt, wenn das Gewichtsverhältnis der (insbesondere festen) Fettalkohole mit 12 bis 18 Kohlenstoffatomen zu den übrigen Fettalkoholen in einem Gewichtsverhältnisbereich von 1 zu 1 bis 1 zu 1.3 liegt.

Davon zunächst unabhängig ist es bevorzugt, wenn das Gewichtsverhältnis der (insbesondere festen) Fettalkohole mit 20 bis 28 Kohlenstoffatomen zu den Fettalkoholen mit 30 bis 38 Kohlenstoffatomen in einem Gewichtsverhältnisbereich von 1 zu 0,5 bis 1 zu 2, insbesondere von 1 zu 0,9 bis 1 zu 0,6 liegt.

Davon zunächst unabhängig ist es bevorzugt, wenn das Gewichtsverhältnis der (insbesondere festen) Fettalkohole mit 20 bis 28 Kohlenstoffatomen zu den Fettalkoholen mit 40 bis 48 Kohlenstoffatomen in einem Gewichtsverhältnisbereich von 1 zu 0,1 bis 1 zu 1, insbesondere von 1 zu 0,2 bis 1 zu 0,4 liegt.

Es ist wiederum erfindungsgemäß besonders bevorzugt, wenn
(i) das Gewichtsverhältnis der (insbesondere festen) Fettalkohole mit 12 bis 18 Kohlenstoffatomen zu den übrigen Fettalkoholen in einem Gewichtsverhältnisbereich von 1 zu 1 bis 1 zu 3, insbesondere von 1 zu 1 bis 1 zu 1.3, liegt,
   und
(ii) das Gewichtsverhältnis der (insbesondere festen) Fettalkohole mit 20 bis 28 zu den Fettalkoholen mit mehr als 28 Kohlenstoffatomen wie folgt verteilt ist:
   (ii-1) das Gewichtsverhältnis der (insbesondere festen) Fettalkohole mit 20 bis 28 Kohlenstoffatomen zu den Fettalkoholen mit 30 bis 38 Kohlenstoffatomen in einem Gewichtsverhältnisbereich von 1 zu 0,5 bis 1 zu 2, insbesondere von 1 zu 0,9 bis 1 zu 0,6 liegt,
      und/oder
   (ii-2) das Gewichtsverhältnis der (insbesondere festen) Fettalkohole mit 20 bis 28 Kohlenstoffatomen zu den Fettalkoholen mit 40 bis 48 Kohlenstoffatomen in einem Gewichtsverhältnisbereich von 1 zu 0,1 bis 1 zu 1, insbesondere von 1 zu 0,2 bis 1 zu 0,4 liegt.

Weitere bevorzugte erfindungsgemäße Zusammensetzungen sind dadurch gekennzeichnet, dass sie, beispielsweise zur Verbesserung der Konsistenz und der sensorischen Eigenschaften zusätzlich mindestens einen festen, wasserunlöslichen teilchenförmigen Füllstoff enthalten. In einer außerordentlich bevorzugten Ausführungsform ist dieser Füllstoff ausgewählt aus gegebenenfalls modifizierten Stärken (z. B. aus Mais, Reis, Kartoffeln) und Stärkederivaten, die gewünschtenfalls vorverkleistert sind (z.B. Dry FLO PC der Firma AKZO), Siliciumdioxid, Kieselsäuren, z. B. Aerosil^{®}-Typen, sphärischen Polyalkylsesquisiloxan-Partikeln (insbesondere Aerosil^{®} R972 und Aerosil^{®} 200V von Degussa), Kieselgelen, Talkum, Kaolin, Magnesiumaluminiumsilikaten, Bornitrid, Lactoglobulinderivaten, z. B. Natrium-C₈₋₁₆-Isoalkylsuccinyllactoglobulinsulfonat, von Brooks Industries erhältlich als Handelsprodukt Biopol^{®} OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen.

Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere Polymerpulver, die sich als erfindungsgemäß bevorzugte Füllstoffe eignen, sind z. B. Polymethacrylate (Micropearl^{®} M von SEPPIC oder Plastic Powder A von NIKKOL), Styrol-Divinylbenzol-Copolymeren (Plastic Powder FP von NIKKOL), Polyethylen- und Polypropylen-Pulver (ACCUREL^{®} EP 400 von AKZO) oder auch Siliconpolymere (Silicone Powder X2-1605 von Dow Corning).

Bevorzugte erfindungsgemäße Zusammensetzungen sind dadurch gekennzeichnet, dass sie mindestens einen festen, wasserunlöslichen teilchenförmigen Füllstoff in einer Gesamtmenge von 1 bis 99 Gew.-%, bevorzugt 2 - 90 Gew.-%, besonders bevorzugt 3 - 15 Gew.-%, außerordentlich bevorzugt 4, 5, 6, 7, 8, 9, 10, 11, 12, 13 und 14 Gew.-%, jeweils bezogen auf die Gesamtzusammensetzung, enthalten.

Weitere bevorzugte erfindungsgemäße Zusammensetzungen sind daher dadurch gekennzeichnet, dass sie zusätzlich mindestens ein lipophiles Verdickungsmittel enthalten.

Erfindungsgemäß bevorzugte lipophile Verdickungsmittel sind ausgewählt aus hydrophobierten Tonmineralien, pyrogenen Kieselsäuren, Ethylene/Propylene/Styrene Copolymeren, Butylene/ Ethylene/Styrene Copolymeren, Dextrinestern, Siliconelastomeren, unter Normalbedingungen festen Wachsen und/oder Glycerintriestern. Besonders bevorzugt enthält die erfindungsgemäße Zusammensetzung als lipophiles Verdickungsmittel zusätzlich mindestens ein Silikonelastomer.

Bevorzugte hydrophobierte Tonmineralien sind ausgewählt aus hydrophobierten Montmorilloniten, hydrophobierten Hectoriten und hydrophobierten Bentoniten, besonders bevorzugt aus Disteardimonium Hectorite, Stearalkonium Hectorite, Quaternium-18 Hectorite und Quaternium-18 Bentonite. Die handelsüblichen Verdickungsmittel stellen diese hydrophobierten Tonmineralien in Form eines Gels in einer Ölkomponente, bevorzugt in Cyclomethicone und/oder einer Nichtsilicon-Ölkomponente, wie z. B. Propylencarbonat, bereit. Derartige Gele sind beispielsweise unter der Handelsbezeichnung Bentone^{®} oder Thixogel erhältlich.

Erfindungsgemäß bevorzugte Zusammensetzungen sind dadurch gekennzeichnet, dass sie mindestens ein hydrophobiertes Tonmineral in einer Gesamtmenge von 0,5 - 10 Gew.-%, bevorzugt 1 - 7 Gew.-%, besonders bevorzugt 2 - 6 Gew.-%, außerordentlich bevorzugt 3 - 5 Gew.-%, jeweils bezogen auf das Gesamtgewicht der erfindungsgemäßen Zusammensetzung, enthalten.

Weitere erfindungsgemäß bevorzugte lipophile Verdickungsmittel sind ausgewählt aus pyrogenen Kieselsäuren, z. B. den Handelsprodukten der Aerosil^{®}-Serie von Evonik Degussa. Besonders bevorzugt sind hydrophobierte pyrogene Kieselsäuren, besonders bevorzugt Silica Silylate und Silica Dimethyl Silylate.

Erfindungsgemäß bevorzugte Zusammensetzungen sind dadurch gekennzeichnet, dass sie mindestens eine pyrogene Kieselsäure, bevorzugt mindestens eine (bevorzugt hydrophobierte) pyrogene Kieselsäure, in einer Gesamtmenge von 0,5 - 10 Gew.-%, bevorzugt 1 - 7 Gew.-%, besonders bevorzugt 2 - 6 Gew.-%, außerordentlich bevorzugt 3 - 5 Gew.-%, jeweils bezogen auf das Gesamtgewicht der erfindungsgemäßen Zusammensetzung, enthalten.

Weitere erfindungsgemäß bevorzugte Zusammensetzungen sind dadurch gekennzeichnet, dass sie mindestens eine hydrophobierte pyrogene Kieselsäure und mindestens eine hydrophile Kieselsäure enthalten.

Weitere erfindungsgemäß bevorzugte lipophile Verdickungsmittel sind ausgewählt aus Ethylene/ Propylene/Styrene Copolymeren und Butylene/Ethylene/Styrene Copolymeren. Besonders bevorugt werden die Copolymere als vorverdicktes öl-basiertes Gel eingesetzt. Derartige Gele sind beispielsweise unter der Handelsbezeichnung Versagel^{®} (ex Penreco) erhältlich. Bevorzugt sind Gele mit Mineralöl, hydriertem Polyisobuten, Isoparaffinen, wie Isohexadecan oder Isododecan, sowie mit Esterölen, insbesondere mit Isopropylpalmitat oder Isopropylmyristat.

Erfindungsgemäß bevorzugte Zusammensetzungen sind dadurch gekennzeichnet, dass sie mindestens ein Ethylene/Propylene/Styrene Copolymer und/oder Butylene/Ethylene/Styrene Copolymer in einer Gesamtmenge von 0,05 - 3 Gew.-%, bevorzugt 0,1 - 2 Gew.-%, besonders bevorzugt 0,2 - 1,0 Gew.-%, außerordentlich bevorzugt 0,3 - 0,5 Gew.-%, jeweils bezogen auf das Gesamtgewicht der erfindungsgemäßen Zusammensetzung, enthalten.

Weitere erfindungsgemäß bevorzugte lipophile Verdickungsmittel sind ausgewählt aus Siliconelastomeren. Eine weitere bevorzugte Ausführungsform der Erfindung ist dadurch gekennzeichnet, dass mindestens ein Siliconelastomer, das erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist, enthalten ist.

Erfindungsgemäß besonders bevorzugte Organopolysiloxane mit mindestens 2 C₂ - C₁₀ - AlkenylGruppen mit terminaler Doppelbindung im Molekül sind ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

Erfindungsgemäß besonders bevorzugte vernetzende Organopolysiloxane mit mindestens zwei Silicon-gebundenen Wasserstoffatomen sind ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-siloxan-Copolymeren.

Erfindungsgemäß besonders bevorzugte Siliconelastomere, die als Rohstoff bereits in einem bei Raumtemperatur unter Normalbedingungen flüssigen Silicon vorgequollen vorliegen und ein Silicon-basiertes Gel darstellen, sind kommerziell erhältlich, beispielsweise unter den Handelsnamen Dow Corning 9040 Silicone Elastomer Blend (ein Cyclomethicone (and) Dimethicone Crosspolymer von Dow Corning; Silikonelastomergehalt 12 - 13 Gew.-%), SFE 168, ein Cyclomethicone (and) Dimethicone/Vinyl Dimethicone Crosspolymer von GE Silicones, Vinyl Dimethicone Crosspolymere, enthalten in KSG-15 (Cyclomethicone (and) Dimethicone/Vinyl Dimethicone Crosspolymer, Siliconelastomergehalt 4 - 10 Gew.-%), KSG-16 (Dimethicone (and) Dimethicone/Vinyl Dimethicone Crosspolymer, Siliconelastomergehalt 20 - 30 Gew.-%), KSG-17 (Cyclomethicone (and) Dimethicone/Vinyl Dimethicone Crosspolymer), KSG-18 (Phenyl Trimethicone (and) Dimethicone/Phenyl Vinyl Dimethicone Crosspolymer, Siliconelastomergehalt 10 - 20 Gew.-%); and KSG-20, erhältlich von Shin Etsu Silicones of America (Akron, Ohio), und von Grant Industries Inc. (Elmwood Park, NJ) die Produkte aus der Gransil^{®}-Serie, insbesondere Gransil SR-CYC (Cyclomethicone and Stearyl-Vinyl/hydromethylsiloxane Copolymer), Gransil^{®} RPS Gel (INCI-Bezeichnung: Cyclopentasiloxane and Polysilicone-11), Gransil^{®} GCM-4 (INCI-Bezeichnung: Cyclotetrasiloxane and Polysilicone-11), Gransil^{®}GCM-5 (INCI-Bezeichnung: Cyclopentasiloxane and Polysilicone-11), Gransil^{®} RPS (INCI-Bezeichnung: Cyclopentasiloxane and Polysilicone-11), GI-CD 10 (INCI-Bezeichnung: Cyclopentasiloxane (and) Stearoxymethicone/Dimethicone Copolymer (and) Dimethicone), Gransil^{®} IDS (INCI-Bezeichnung: Isododecane (and) Cyclotetrasiloxane (and) Polysilicone-11), Gransil^{®}PC-12 (INCI-Bezeichnung: Isododecane (and) Polysilicone-11), Gransil^{®}IDS-5 (INCI-Bezeichnung: Isododecane (and) Cyclopentasiloxane (and) Polysilicone-11), Gransil^{®}APK-1 (INCI-Bezeichnung: Dimethicone and Cyclopentasiloxane and Polysilicone-11 and Nylon-12 and Methyl Methacrylate/Acrylonitrile Copolymer and PEG-10 Dimethicone and Polysorbate-40 and Isohexadecane and Ammonium Polyacryloyldimethyl Taurate), Gransil^{®}DMCM-5 (INCI-Bezeichnung: Dimethicone and Cyclopentasiloxane and Polysilicone-11), Gransil^{®}DMG-6 mit Dimethicone (6 cSt) (INCI-Bezeichnung: Dimethicone and Polysilicone-11), Gransil^{®}DMG-20 mit Dimethicone (20 cSt) (INCI-Bezeichnung: Dimethicone and Polysilicone-11), Gransil^{®} AM-8 Gel (INCI-Bezeichnung: Caprylyl Methicone and Cyclopentasiloxane and Polysilicone-11), Gransil^{®} DM 5 mit Dimethicone (5 cSt) (INCI-Bezeichnung: Dimethicone and Polysilicone-11), Gransil^{®} DMID (INCI-Bezeichnung: Dimethicone and Isododecane and Polysilicone-11), Gransil^{®} PM (INCI-Bezeichnung: Phenyl Trimethicone and Polysilicone-11), Gransil^{®} ININ (INCI-Bezeichnung: Isononyl Isononanoate (and) Polysilicone-11).

Ebenfalls mit Vorzug in den erfindungsgemäßen Zusammensetzungen einsetzbar sind Siliconelastomere, die als Rohstoff bereits in einem bei Raumtemperatur unter Normalbedingungen flüssigen Silicon, gemischt mit einem Nicht-Silicon-haltigen Öl, Fett oder Wachs, vorgequollen vorliegen und ein Silicon-/Nicht-Silicon-basiertes Gel darstellen. Derartige Siliconelastomer-Zusammensetzungen sind ebenfalls kommerziell erhältlich, beispielsweise unter den Handelsnamen Gransil^{®} MLB (INCI-Bezeichnung: Cyclopentasiloxane and Polysilicone-11 and Beeswax), Gransil^{®} PS (INCI-Bezeichnung: Cyclotetrasiloxane and Polysilicone-11 and Petrolatum), Gransil^{®} PS-5 (INCI-Bezeichnung: Cyclopentasiloxane and Polysilicone-11 and Petrolatum), Gransil^{®} DMG-20 P mit Dimethicone (20 cSt) und Petrolatum (INCI-Bezeichnung: Dimethicone and Polysilicone-11 and Petrolatum), Gransil^{®} RJO (INCI-Bezeichnung: Cyclopentasiloxane and Polysilicone-11 and Jojoba Oil), Gransil^{®} LANO (INCI-Bezeichnung: Cyclopentasiloxane and Polysilicone-11 and Lanolin), Gransil^{®} OHS-5 (INCI-Bezeichnung: Cyclopentasiloxane and Polysilicone-11 and Octyl Hydroxystearate) und Gransil^{®} DML (INCI-Bezeichnung: Dimethicone (and) Neopentyl Glycol Diheptanoate (and) Polysilicone-11).

Eine weitere bevorzugte Ausführungsform der Erfindung ist dadurch gekennzeichnet, dass das Siliconelastomer durch die Vernetzung eines Organopolysiloxans, das mindestens 2 C₂ - C₁₀-Alkenylgruppen mit terminaler Doppelbindung in jedem Molekül enthält, mit mindestens einem alpha, omega-Dien erhältlich ist. Erfindungsgemäß besonders bevorzugte vernetzende alpha, omega-Diene weisen die Formel CH₂=CH(CH₂)ₓCH=CH₂ mit x = 1 - 20 auf. Besonders bevorzugte alpha, omega-Diene sind ausgewählt aus 1,4-Pentadien, 1,5-Hexadien, 1,6-Heptadien, 1,7-Octadien, 1,8-Nonadien, 1,11-Dodecadien, 1,13-Tetradecadien und 1,19- Eicosadien.

Erfindungsgemäß bevorzugte Zusammensetzungen sind dadurch gekennzeichnet, dass sie mindestens ein Siliconelastomer in einer Gesamtmenge von 0,05 - 3 Gew.-%, bevorzugt 0,1 - 2 Gew.-%, besonders bevorzugt 0,2 - 1,0 Gew.-%, außerordentlich bevorzugt 0,3 - 0,5 Gew.-%, jeweils bezogen auf das Gesamtgewicht der erfindungsgemäßen Zusammensetzung, enthalten.

Als ein bei Normalbedingungen festes Wachs enthält das erfindungsgemäße Mittel bevorzugt zusätzlich mindestens ein Polyethylenwachs. Bevorzugt geeignete Polyethylenwachse weisen 30 bis 60 Kohlenstoffatome auf.

Weitere bevorzugte erfindungsgemäße Zusammensetzungen sind dadurch gekennzeichnet, dass sie mindestens einen Duftstoff und/oder mindestens ein Parfümöl enthalten.

Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Zu den phenolischen Riechstoffverbindungen zählt z. B. Carvacrol. Riechstoffverbindungen vom Typ der Ester sind z.B. Benzylacetat, Methylanthranilat, ortho-t-Butylcyclohexylacetat, p-tert.-Butylcyclohexylacetat, Diethylphthalat, Nonandiol-1,3-diacetat, iso-Nonylacetat, iso-Nonylformiat, Phenylethylphenylacetat, Phenoxyethylisobutyrat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat, Benzylsalicylat, Ethylsalicylat, iso-Amylsalicylat, Hexylsalicylat und 4-Nonanolid. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. 6-Acetyl-1,1,3,4,4,6-hexamethyltetrahydronaphthalin, para-t-Amylcyclohexanon, 2-n-Heptylcyclopentanon, β-Methylnaphthylketon und die lonone α-Isomethylionon und Methylcedrylketon, zu den Alkoholen Zimtalkohol, Anethol, Citronellol, Dimyrcetol, Eugenol, Geraniol, Linalool, Phenylethylalkohol und Terpineol, zu den Kohlenwasserstoffen gehören 1,3,4,6,7,8-Hexahydro-4,6,6,7,8,8-hexamethylcyclopenta-a-2-benzopyran, Hydroxymethylisopropylcyclopentan, 3-a-Methyldodecahydro-6,6,9a-trimethylnaphtho-2(2,1-b)furan, iso-Butylchinolin sowie die Terpene und Balsame. Bevorzugt werden Mischungen verschiedener Riechstoffe verwendet, die gemeinsam eine ansprechende Duftnote erzeugen.

Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, iso-Eugenol, Thymianöl, Bergamotteöl, Geraniumöl und Rosenöl.

Bevorzugte erfindungsgemäße Zusammensetzungen enthalten mindestens einen Duftstoff und/ oder mindestens ein Parfümöl in einer Gesamtmenge von 0,01 - 10 Gew.-%, bevorzugt 0,1 - 5 Gew.-%, besonders bevorzugt 0,5 - 3 Gew.-%, außerordentlich bevorzugt 1 - 2 Gew.-%, jeweils bezogen auf das Gesamtgewicht der erfindungsgemäßen Zusammensetzung.

Bevorzugte erfindungsgemäße Zusammensetzungen sind dadurch gekennzeichnet, dass sie zusätzlich mindestens einen Deodorant-Wirkstoff enthalten. Bevorzugte derartige Deodorant-Wirkstoffe sind ausgewählt aus Geruchsabsorbern, desodorierend wirkenden Ionenaustauschern, keimhemmenden Substanzen, präbiotisch wirksamen Substanzen sowie Enzyminhibitoren und, besonders bevorzugt, Kombinationen der genannten Deodorant-Wirkstoffe.

Silicate dienen als Geruchsabsorber, die auch gleichzeitig die rheologischen Eigenschaften der erfindungsgemäßen Zusammensetzung vorteilhaft unterstützen. Zu den erfindungsgemäß besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll.

Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Weiterhin sind Phenol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.

Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.

Zu den Enzyminhibitoren gehören Stoffe, die die für die Schweißzersetzung verantwortlichen Enzyme, insbesondere die Arylsulfatase, β-Glucuronidase, Aminoacylase, Esterasen, Lipasen und/oder Lipoxigenase, hemmen, z. B. Trialkylcitronensäureester, insbesondere Triethylcitrat, oder Zinkglycinat.

Bevorzugte erfindungsgemäße Zusammensetzungen sind dadurch gekennzeichnet, dass der mindestens eine zusätzliche Deodorant-Wirkstoff ausgewählt ist aus Arylsulfatase-Inhibitoren, β-Glucuronidase-Inhibitoren, Aminoacylase-Inhibitoren, Esterase-Inhibitoren, Lipase-Inhibitoren und Lipoxigenase-Inhibitoren, α-Monoalkylglycerinethern mit einem verzweigten oder linearen gesättigten oder ungesättigten, gegebenenfalls hydroxylierten C₆ - C₂₂-Alkylrest, insbesondere α-(2-Ethylhexyl)glycerinether, präbiotisch wirksamen Komponenten, Trialkylcitronensäureestern, insbesondere Triethylcitrat, Wirkstoffen, die die Zahl der an der Geruchsbildung beteiligten Hautkeime aus der Gruppe der Staphylokokken, Corynebakterien, Anaerokokken und Mikrokokken reduzieren bzw. deren Wachstum hemmen, Zinkverbindungen, insbesondere Zinkphenolsulfonat und Zinkricinoleat, Organohalogenverbindungen, insbesondere Triclosan, Chlorhexidin, Chlorhexidingluconat und Benzalkoniumhalogeniden, quartären Ammoniumverbindungen, insbesondere Cetylpyridiniumchlorid, Geruchsabsorbern, insbesondere Silikaten und Zeolithen, Natriumbicarbonat, Lantibiotika, sowie Mischungen der vorgenannten Substanzen.

Weitere bevorzugte erfindungsgemäße Zusammensetzungen sind dadurch gekennzeichnet, dass der mindestens eine zusätzliche Deodorant-Wirkstoff in einer Gesamtmenge von 0,1 - 10 Gew.-%, bevorzugt 0,2 - 7 Gew.-%, besonders bevorzugt 0,3 - 5 Gew.-%, bezogen auf das Gesamtgewicht der Aktivsubstanz in der Gesamtzusammensetzung, enthalten ist.

Antioxidative Stoffe können der oxidativen Zersetzung der Schweißkomponenten entgegenwirken und auf diese Weise die Geruchsentwicklung hemmen. Geeignete Antioxidantien sind Imidazol und Imidazolderivate (z. B. Urocaninsäure), Peptide wie z. B. D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis µmol/kg), ferner Metallchelatoren (z. B. α-Hydroxyfettsäuren, EDTA, EGTA, Lactoferrin), Huminsäuren, Gallensäure, Gallenextrakte, Catechine, Bilirubin, Biliverdin und deren Derivate, ungesättigte Fettsäuren und deren Derivate (z. B. γ-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -stearat, - dipalmitat, -acetat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivate, Tocopherole und deren Derivate (z. B. Tocopherylacetat, -linoleat, -oleat und - succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate, Kojisäure, Chitosanglycolat und -salicylat, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Selen und Selen-Derivate (z. B. Selenmethionin), Stilbene und Stilben-Derivate (z. B. Stilbenoxid, trans-Stilbenoxid). Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte, die diese Antioxidantien enthalten, eingesetzt werden.

Als lipophile, öllösliche Antioxidantien aus dieser Gruppe sind Tocopherol und dessen Derivate, insbesondere Tocopherylacetat, und Carotinoide sowie Butylhydroxytoluol/anisol bevorzugt.

Die Gesamtmenge der Antioxidantien in bevorzugten erfindungsgemäßen Zubereitungen beträgt 0,001 - 10 Gew.-%, vorzugsweise 0,05 - 5 Gew.-% und insbesondere 0,1 - 2 Gew.-%, bezogen auf die gesamte Zubereitung.

Auch komplexbildende Stoffe können die deodorierende Wirkung unterstützen, indem sie die oxidativ katalytisch wirkenden Schwermetallionen (z. B. Eisen oder Kupfer) stabil komplexieren. Geeignete Komplexbildner sind ausgewählt aus den vorstehend genannten Komplexbildnern.

Ein zweiter Gegenstand der Offenbarung ist ein kosmetisches Produkt, umfassend
i) eine Abgabevorrichtung, umfassend
   - mindestens einen Behälter, der einen Kolben enthält, welcher dem Behälter als Wandung dient und sich durch einen Abgabemechanismus (insbesondere eine Mutter-Spindel-Anordnung) bewegen lässt,
   - mindestens eine Austrittsöffnung, die in Fluidverbindung mit dem besagten Behälter steht,
ii) eine in dem besagten Behälter befindliche Zusammensetzung des ersten Erfindungsgegenstandes.

Die Zusammensetzung des ersten Erfindungsgegenstandes lässt sich durch Betätigung des Abgabemechanismusses aus der Austrittsöffnung der Abgabevorrichtung ausbringen. Dabei wird der Kolben bewegt und die Zusammensetzung des ersten Erfindungsgegenstandes in Richtung der Austrittsöffnung gedrückt. Durch den dabei entstehenden Druck, wird die besagte Zusammensetzung durch die mindestens eine Austrittsöffnung aus der besagten Abgabevorrichtung hinaus getrieben.

Ein dritter Gegenstand der vorliegenden Offenbarung ist die nicht-therapeutische Verwendung der Zusammensetzungen des ersten Erfindungsgegenstandes zur Reduzierung oder Maskierung von Körpergeruch.

Ein vierter Gegenstand der vorliegenden Offenbarung ist ein nicht-therapeutisches Verfahren zur Reduzierung oder Maskierung von Körpergeruch, das dadurch gekennzeichnet ist, dass eine Zusammensetzung des ersten Erfindungsgegenstandes in einer wirksamen Menge mit einem geeigneten Applikator auf die Haut aufgetragen wird. Besonders bevorzugt verbleibt die erfindungsgemäße Zusammensetzung für eine Zeit von 1 Minute bis 24 Stunden, bevorzugt 2 - 12 Stunden, auf der Haut.

Eine besonders bevorzugte Ausführungsform der Offenbarung wird durch folgende Punkte charakterisiert:
1. Wasserfreie Zusammensetzung, die jeweils bezogen auf das Gesamtgewicht der Zusammensetzung enthält
   - mindestens einen Antitranspirant-Wirkstoff,
   - mindestens ein Öl in einer Gesamtmenge von 20 bis 80 Gew.-%,
   - mindestens einen Fettalkohol mit 12 bis 18 Kohlenstoffatomen,
   - mindestens einen Fettalkohol mit mehr als 18 Kohlenstoffatomen,
   mit der Maßgabe, dass
   - die Gesamtmenge an Fettalkoholen 3 bis 12 Gew.-% beträgt,
   - das Gewichtsverhältnis der Fettalkohole mit 12 bis 18 Kohlenstoffatomen zu den übrigen Fettalkoholen in einem Gewichtsverhältnisbereich von 1 zu 1 bis 1 zu 3 liegt.
2. Wasserfreie Zusammensetzung nach Punkt 1, dadurch gekennzeichnet, dass der mindestens eine Antitranspirant-Wirkstoff in einer Menge von 3 bis 35 Gew.-%, bevorzugt 5 bis 30 Gew.-% und besonders bevorzugt 10 bis 23 Gew.-%, enthalten ist, bezogen auf das Gesamtgewicht der kristallwasserfreien Aktivsubstanz (USP) in der Gesamtzusammensetzung.
3. Wasserfreie Zusammensetzung nach einem der Punkte 1 oder 2, dadurch gekennzeichnet, dass sie die Öle in einer Gesamtmenge von 30 bis 80 Gew.-%, besonders bevorzugt von 40 bis 75 Gew.-%, ganz besonders bevorzugt von 45 bis 70 Gew.%, enthält.
4. Wasserfreie Zusammensetzung nach einem der Punkte 1 bis 3, dadurch gekennzeichnet, dass als Öl mindestens ein flüchtiges Öl enthalten ist.
5. Wasserfreie Zusammensetzung nach Punkt 4, dadurch gekennzeichnet, dass als flüchtiges Öl mindestens ein flüchtiges Siliconöl und/oder mindestens ein C₈-C₁₆-Isoparaffinen enthalten ist.
6. Wasserfreie Zusammensetzung nach einem der Punkte 1 bis 5, dadurch gekennzeichnet, dass die Gesamtmenge an Fettalkoholen 4 bis 9 Gew.-% beträgt.
7. Wasserfreie Zusammensetzung nach einem der Punkte 1 bis 6, dadurch gekennzeichnet, dass bezogen auf ihr Gesamtgewicht die Fettalkohole mit 18 Kohlenstoffatomen in einer Gesamtmenge von 2,0 bis 5,0 Gew.-% enthalten sind.
8. Wasserfreie Zusammensetzung nach einem der Punkte 1 bis 7, dadurch gekennzeichnet, dass bezogen auf ihr Gesamtgewicht die Fettalkohole mit 20 bis 28 Kohlenstoffatomen in einer Gesamtmenge von 0,8 bis 2,0 Gew.-% enthalten sind.
9. Wasserfreie Zusammensetzung nach einem der Punkte 1 bis 8, dadurch gekennzeichnet, dass bezogen auf ihr Gesamtgewicht die Fettalkohole mit 30 bis 38 Kohlenstoffatomen in einer Gesamtmenge von 0,6 bis 1,5 Gew.-% enthält.
10. Wasserfreie Zusammensetzung nach einem der Punkte 1 bis 9, dadurch gekennzeichnet, dass bezogen auf ihr Gesamtgewicht die Fettalkohole mit 40 bis 48 Kohlenstoffatomen in einer Gesamtmenge von 0,2 bis 0,7 Gew.-% enthält.
11. Wasserfreie Zusammensetzung nach einem der Punkte 1 bis 10, dadurch gekennzeichnet, dass bezogen auf ihr Gesamtgewicht weniger als 0,05 Gew.-% Fettalkohole mit mehr als 50 Kohlenstoffatomen enthalten sind.
12. Wasserfreie Zusammensetzung nach einem der Punkte 1 bis 11, dadurch gekennzeichnet, dass das Gewichtsverhältnis der Fettalkohole mit 12 bis 18 Kohlenstoffatomen zu den übrigen Fettalkoholen in einem Gewichtsverhältnisbereich von 1 zu 1 bis 1 zu 1,3 liegt.
13. Wasserfreie Zusammensetzung nach einem der Punkte 1 bis 12, dadurch gekennzeichnet, dass das Gewichtsverhältnis der Fettalkohole mit 20 bis 28 Kohlenstoffatomen zu den Fettalkoholen mit 30 bis 38 Kohlenstoffatomen in einem Gewichtsverhältnisbereich von 1 zu 0,5 bis 1 zu 2, insbesondere von 1 zu 0,9 bis 1 zu 0,6 liegt.
14. Wasserfreie Zusammensetzung nach einem der Punkte 1 bis 13, dadurch gekennzeichnet, dass das Gewichtsverhältnis der Fettalkohole mit 20 bis 28 Kohlenstoffatomen zu den Fettalkoholen mit 40 bis 48 Kohlenstoffatomen in einem Gewichtsverhältnisbereich von 1 zu 0,1 bis 1 zu 1, insbesondere von 1 zu 0,2 bis 1 zu 0,4 liegt.
15. Wasserfreie Zusammensetzung nach einem der Punkte 1 bis 14, dadurch gekennzeichnet, dass sie zusätzlich mindestens ein lipophiles Verdickungsmittel, insbesondere vom Typ der Silicon Elastomere enthält.
16. Nicht-therapeutische Verwendung einer Zusammensetzung gemäß einem der Punkte 1 bis 15 zur Reduzierung oder Maskierung von Körpergeruch.
17. Nicht-therapeutisches Verfahren zur Reduzierung oder Maskierung von Körpergeruch, dadurch gekennzeichnet, dass eine Zusammensetzung gemäß einem der Punkte 1 bis 15 in einer wirksamen Menge mit einem geeigneten Applikator auf die Haut aufgetragen wird.
18. Kosmetisches Produkt, umfassend
   i) eine Abgabevorrichtung, umfassend
      - mindestens einen Behälter, der einen Kolben enthält, welcher dem Behälter als Wandung dient und sich durch einen Abgabemechanismus (insbesondere eine Mutter-Spindel-Anordnung) bewegen lässt,
      - mindestens eine Austrittsöffnung, die in Fluidverbindung mit dem besagten Behälter steht,
   ii) eine in dem besagten Behälter befindliche Zusammensetzung gemäß einem der Punkte 1 bis 15.

Die nachfolgenden Beispiele sollen die Erfindung verdeutlichen, ohne sie hierauf zu beschränken. Alle Mengenangaben beziehen sich, soweit nicht anders vermerkt, auf Gew.-% bezogen auf das Gesamtgewicht der entsprechenden Zusammensetzung.

### Beispiele

Folgende soft solid-Zusammensetzungen wurden hergestellt:

| **Rohstoff** | **A** | **B** | **C** | **D** | **E** | **F** | **G** |
|---|---|---|---|---|---|---|---|
| Dow Corning 9040 Silicone Elastomer Blend | 5,0 | 5,0 | 5,0 | 5,0 | 5,0 | 5,0 | 5,0 |
| Cyclopentasiloxane | 69,5 | 65,5 | 51,5 | - | 66,5 | 32,75 | 65,5 |
| C10-13 Isoparaffin | - | - | - | 51,5 | - | 32,75 | - |
| Aerosil 300 ¹ | 0,5 | 0,5 | 0,5 | 0,5 | 0,5 | 0,5 | 0,5 |
| Stearylalkohol | 2,1 | 3,8 | 2,1 | 2,1 | 3,8 | 3,8 | 3,8 |
| Performacol 425 Alcohol ² | 2,9 | 5,2 | 2,9 | 2,9 | 5,2 | 5,2 | 5,2 |
| Dry Flo PC ³ | - | - | 6,5 | 6,5 | - | - | - |
| Talkum | - | - | 11,9 | 11,9 | - | - | - |
| Aluminium Zirconium Pentachlorohydrex Gly (AAZG 3110) | 20,0 | 20,0 | 20,0 | 20,0 | - | - | - |
| Aluminium Zirconium Trichlorohydrex Gly (AAZG 531) | - | - | - | - | 23,0 | - | 20,0 |
| Aluminium Chlorohydrate (Microdry 3110) | - | - | - | - | - | 20,0 | - |

| | | | | | | | |
|---|---|---|---|---|---|---|---|
| 1 INCI-Bezeichnung: Silica (pyrogenes Silika, mittlere Partikelgröße: 7 µm, Evonik) 2 INCI-Bezeichnung: C20-40 Alcohols (Firma Baker Petrolite) 3 INCI-Bezeichnung: Aluminium Starch Octenylsuccinate (hydrophob modifizierte Maisstärke mit einem Molekulargewicht von 10000, AKZO) | | | | | | | |

Es wurde der Antitranspiranswirkstoff in Cyclopentasiloxan mit einem Silikonelastomer und Silika suspendiert. Die optionalen weiteren Komponenten Dry Fo PC und Talkum wurden ebenso suspendiert. Danach wurden die Fettalkohole unter Rühren dazugegeben, bis sich eine verdicktes soft solid gebildet hatte.

Die Zusammensetzungen waren stabil und zeigten auch unter Druck keine Synerese. Die Rezepturen wiesen ein leichtes, puderig-trockenes Gefühl bei der Applikation auf die Haut auf.

## Patentansprüche

1. Wasserfreie Zusammensetzung, die jeweils bezogen auf das Gesamtgewicht der Zusammensetzung enthält
- mindestens einen Antitranspirant-Wirkstoff,
- mindestens ein Öl in einer Gesamtmenge von 20 bis 80 Gew.-%,
- mindestens einen Fettalkohol mit 12 bis 18 Kohlenstoffatomen,
- mindestens einen Fettalkohol mit mehr als 18 Kohlenstoffatomen,
mit der Maßgabe, dass
- die Gesamtmenge an Fettalkoholen 3 bis 12 Gew.-% beträgt,
- das Gewichtsverhältnis der Fettalkohole mit 12 bis 18 Kohlenstoffatomen zu den übrigen Fettalkoholen in einem Gewichtsverhältnisbereich von 1 zu 1 bis 1 zu 1,3 liegt.

2. Wasserfreie Zusammensetzung nach Anspruch 1, **dadurch gekennzeichnet, dass** der mindestens eine Antitranspirant-Wirkstoff in einer Menge von 3 bis 35 Gew.-%, bevorzugt 5 bis 30 Gew.-% und besonders bevorzugt 10 bis 23 Gew.-%, enthalten ist, bezogen auf das Gesamtgewicht der kristallwasserfreien Aktivsubstanz (USP) in der Gesamtzusammensetzung.

3. Wasserfreie Zusammensetzung nach einem der Ansprüche 1 oder 2, **dadurch gekennzeichnet, dass** sie die Öle in einer Gesamtmenge von 30 bis 80 Gew.-%, besonders bevorzugt von 40 bis 75 Gew.-%, ganz besonders bevorzugt von 45 bis 70 Gew.%, enthält.

4. Wasserfreie Zusammensetzung nach einem der Ansprüche 1 bis 3, **dadurch gekennzeichnet, dass** als Öl mindestens ein flüchtiges Öl enthalten ist.

5. Wasserfreie Zusammensetzung nach Anspruch 4, **dadurch gekennzeichnet, dass** als flüchtiges Öl mindestens ein flüchtiges Siliconöl und/oder mindestens ein C₈-C₁₆-Isoparaffin enthalten ist.

6. Wasserfreie Zusammensetzung nach einem der Ansprüche 1 bis 5, **dadurch gekennzeichnet, dass** die Gesamtmenge an Fettalkoholen 4 bis 9 Gew.-% beträgt.

7. Wasserfreie Zusammensetzung nach einem der Ansprüche 1 bis 6, **dadurch gekennzeichnet, dass** das Gewichtsverhältnis der Fettalkohole mit 20 bis 28 Kohlenstoffatomen zu den Fettalkoholen mit 30 bis 38 Kohlenstoffatomen in einem Gewichtsverhältnisbereich von 1 zu 0,5 bis 1 zu 2, insbesondere von 1 zu 0,9 bis 1 zu 0,6 liegt.

8. Wasserfreie Zusammensetzung nach einem der Ansprüche 1 bis 7, **dadurch gekennzeichnet, dass** das Gewichtsverhältnis der Fettalkohole mit 20 bis 28 Kohlenstoffatomen zu den Fettalkoholen mit 40 bis 48 Kohlenstoffatomen in einem Gewichtsverhältnisbereich von 1 zu 0,1 bis 1 zu 1, insbesondere von 1 zu 0,2 bis 1 zu 0,4 liegt.

9. Wasserfreie Zusammensetzung nach einem der Ansprüche 1 bis 8, **dadurch gekennzeichnet, dass** sie zusätzlich mindestens ein lipophiles Verdickungsmittel, insbesondere vom Typ der Silicon Elastomere enthält.

## Claims

1. A water-free composition that contains, in each case based on the total weight of the composition,
- at least one antiperspirant substance,
- at least one oil in a total amount of from 20 to 80 wt.%,
- at least one fatty alcohol having 12 to 18 carbon atoms,
- at least one fatty alcohol having more than 18 carbon atoms,
with the proviso that
- the total amount of fatty alcohols is from 3 to 12 wt.%,
- the weight ratio of the fatty alcohols having 12 to 18 carbon atoms to the remaining fatty alcohols is in a weight ratio range of from 1:1 to 1:1.3.

2. The water-free composition according to claim 1, **characterized in that** the at least one antiperspirant substance is contained in an amount of from 3 to 35 wt.%, preferably from 5 to 30 wt.%, and particularly preferably from 10 to 23 wt.%, based on the total weight of the active substance (USP), free from water of crystallization, in the total composition.

3. The water-free composition according to one of claims 1 or 2, **characterized in that** it contains the oils in a total amount of from 30 to 80 wt.%, particularly preferably from 40 to 75 wt.%, very particularly preferably from 45 to 70 wt.%.

4. The water-free composition according to one of claims 1 to 3, **characterized in that** at least one volatile oil is contained as an oil.

5. The water-free composition according to claim 4, **characterized in that** at least one volatile silicone oil and/or at least one C₈-C₁₆ isoparaffin is contained as a volatile oil.

6. The water-free composition according to one of claims 1 to 5, **characterized in that** the total amount of fatty alcohols is from 4 to 9 wt.%.

7. The water-free composition according to one of claims 1 to 6, **characterized in that** the weight ratio of the fatty alcohols having 20 to 28 carbon atoms to the fatty alcohols having 30 to 38 carbon atoms is in a weight ratio range of from 1:0.5 to 1:2, in particular from 1:0.9 to 1:0.6.

8. The water-free composition according to one of claims 1 to 7, **characterized in that** the weight ratio of the fatty alcohols having 20 to 28 carbon atoms to the fatty alcohols having 40 to 48 carbon atoms is in a weight ratio range of from 1:0.1 to 1:1, in particular 1:0.2 to 1:0.4.

9. The water-free composition according to one of claims 1 to 8, **characterized in that** it additionally contains at least one lipophilic thickener, in particular of the type that contains silicone elastomers.

## Revendications

1. Composition anhydre qui contient, respectivement rapporté au poids total de la composition :
- au moins une substance active antitranspirante,
- au moins une huile à hauteur de 20 à 80 % en poids,
- au moins un alcool gras avec 12 à 18 atomes de carbone,
- au moins un alcool gras avec plus de 18 atomes de carbone,
à condition que :
- la quantité totale d'alcools gras est de 3 à 12 % en poids,
- le rapport pondéral des alcools gras avec 12 à 18 atomes de carbone aux alcools gras traditionnels se situe entre 1:1 et 1:1,3.

2. Composition anhydre selon la revendication 1, **caractérisée en ce que** l'au moins une substance active antitranspirante est présente à hauteur de 3 à 35 % en poids, de préférence de 5 à 30 % en poids, et particulièrement préférentiellement de 10 à 23 % en poids, rapporté au poids total de la substance active sans eau de cristallisation (USP) dans la composition totale.

3. Composition anhydre selon la revendication 1 ou 2, **caractérisée en ce qu'**elle contient les huiles à hauteur de 30 à 80 % en poids, particulièrement préférentiellement de 40 à 75 % en poids, tout particulièrement de 45 à 70 % en poids.

4. Composition anhydre selon une des revendications 1 à 3, **caractérisée en ce qu'**elle contient comme huile au moins une huile volatile.

5. Composition anhydre selon la revendication 4, **caractérisée en ce qu'**elle contient comme huile volatile au moins une huile de silicone volatile et/ou une C₈-₁₆-isoparaffine.

6. Composition anhydre selon une des revendications 1 à 5, **caractérisée en ce que** la quantité totale d'alcools gras est de 4 à 9 % en poids.

7. Composition anhydre selon une des revendications 1 à 6, **caractérisée en ce que** le rapport pondéral des alcools gras avec 20 à 28 atomes de carbone aux alcools gras avec 30 à 38 atomes de carbone se situe entre 1:0,5 et 1:2, en particulier entre 1:0,9 et 1:0,6.

8. Composition anhydre selon une des revendications 1 à 7, **caractérisée en ce que** le rapport pondéral des alcools gras avec 20 à 28 atomes de carbone aux alcools gras avec 40 à 48 atomes de carbone se situe entre 1:0,1 et 1:1, en particulier entre 1:0,2 et 1:0,4.

9. Composition anhydre selon une des revendications 1 à 8, **caractérisée en ce qu'**elle contient en plus au moins un épaississant lipophile, en particulier du type des élastomères silicones.
